# EUROPEAN PATENT APPLICATION

(11) **EP 2 716 209 A1**
(43) Date of publication of application: **09.04.2014**
(21) Application number: 13186190.8
(22) Date of filing: 26.09.2013
(51) Int. Cl.: A61B 5/0452, A61B 5/00, G06F 19/00, A61B 5/04, A61N 1/39

(54) **Apparatus for determining possiblity of return of spontaneous circulation**

(30) Priority: 03.10.2012 JP 2012221100
(71) Applicant: Nihon Kohden Corporation, Shinjuku-ku Tokyo (JP); TOKYO MEDICAL UNIVERSITY, Tokyo 160-8402 (JP)
(72) Inventor: Nagata, Shinya, Shinjuku-ku, Tokyo (JP); Oda, Jun, Shinjuku-ku, Tokyo 160-0023 (JP)
(74) Representative: Grünecker, Kinkeldey, Stockmair & Schwanhäusser

(57) **Abstract**

An apparatus for determining a possibility of return of spontaneous circulation, includes: an electrocardiogram acquiring unit acquiring electrocardiogram waveform data from a subject in a cardiac arrest condition during chest compression applied to the subject; a bandpass filter unit allowing only a frequency component in a vicinity of a frequency of a P wave of the electrocardiogram waveform to pass through the bandpass filter unit; a resuscitation possibility determining unit determining whether an output of the bandpass filter unit exceeds a predetermined value or not, and determining that there is the possibility of return of spontaneous circulation when the output exceeds the predetermined value; and an outputting unit outputting a result of determination related to the possibility of return of spontaneous circulation.

## Description

### CROSS-REFERENCE TO RELATED APPLICATION(S)

This application is based upon and claims the benefit of priority from prior Japanese patent application No. 2012-221100, filed on October 10, 2012, the entire contents of which are incorporated herein by reference.

### BACKGROUND

The presently disclosed subject matter relates to an apparatus for determining the possibility of return of spontaneous circulation based on an electrocardiogram during, for example, chest compression.

A system has been proposed which determines whether continuation of applying a resuscitation effort to a patient is preferred or not, based on parameters such as the end-tidal carbon dioxide level, arterial blood pressure, and heart rate of the patient (see Japanese Patent No. 4,430,940).

Also, an apparatus has been proposed which monitors an electrocardiogram waveform of a patient experiencing arrhythmia, and which determines whether defibrillation (DF) is to be performed or not (see JP-T-2008-543479 and JP-A-2005-339533).

In the above-described related art, in a state where the cardiopulmonary function does not stop although it incompletely operates, it is determined whether the resuscitation effort is to be continued or defibrillation (DF) is to be performed. In a cardiopulmonary arrest condition, namely, it cannot be determined whether chest compression is to be continued or not.

### SUMMARY

The presently disclosed subject matter may provide an apparatus which can determine whether there is the possibility that return of spontaneous circulation is enabled by continuing chest compression in a cardiopulmonary arrest condition, or not.

The apparatus for determining a possibility of return of spontaneous circulation may comprise: an electrocardiogram acquiring unit which is configured to acquire electrocardiogram waveform data from a subject in a cardiac arrest condition during chest compression which is applied to the subject as a cardiopulmonary resuscitation process; a bandpass filter unit which is configured to receive the acquired electrocardiogram waveform data, and which is configured to allow only a frequency component in a vicinity of a frequency of a P wave of the received electrocardiogram waveform to pass through the bandpass filter unit; a resuscitation possibility determining unit which is configured to determine whether an output of the bandpass filter unit exceeds a predetermined value or not, and which is configured to determine that there is the possibility of return of spontaneous circulation when the output exceeds the predetermined value; and an outputting unit which is configured to output a result of determination related to the possibility of return of spontaneous circulation.

The frequency component allowed to pass the bandpass filter unit may be a frequency component of 10 Hz to 15 Hz.

There may be also provided a method of determining a possibility of return of spontaneous circulation, the method comprising: acquiring electrocardiogram waveforms data from a subject in a cardiac arrest condition during chest compression which is applied to the subject as a cardiopulmonary resuscitation process; receiving the acquired electrocardiogram waveform data, allowing only a frequency component in a vicinity of a frequency of a P wave of the received electrocardiogram waveform to pass through a bandpass filter; determining whether an output of the bandpass filter exceeds a predetermined value or not, and determining that there is the possibility of return of spontaneous circulation when the output exceeds the predetermined value; and outputting a result of determination related to the possibility of return of spontaneous circulation.

The frequency component allowed to pass the bandpass filter may be a frequency component of 10 Hz to 15 Hz.

There may be also provided an apparatus for determining a possibility of return of spontaneous circulation, the apparatus comprising: an electrocardiogram acquiring unit which is configured to acquire electrocardiogram waveform data from a subject in a cardiac arrest condition during a cardiopulmonary resuscitation process applied to the subject; a resuscitation possibility determining unit which is configured to receive the acquired electrocardiogram waveform data, and which is configured to determine that there is a possibility of return of spontaneous circulation when an amplitude of the received electrocardiogram waveform data is not lower than a reference value; and an outputting unit which is configured to output a result of the determination.

There may be also provided a method of determining a possibility of return of spontaneous circulation, the method comprising: acquiring electrocardiogram waveform data from a subject in a cardiac arrest condition during a cardiopulmonary resuscitation process applied to the subject; receiving the acquired electrocardiogram waveform data, and determining that there is a possibility of return of spontaneous circulation when an amplitude of the received electrocardiogram waveform data is not lower than a reference value; and outputting a result of the determination,

There may be also provided a non-transitory computer-readable recording medium in which a computer program causing a computer to execute the method is recorded.

### BRIEF DESCRIPTION OF THE DRAWINGS

Fig. 1 is a functional block diagram of an apparatus which is a first embodiment of the presently disclosed subject matter, and which determines the possibility of return of spontaneous circulation.
Fig. 2 is a diagram showing the hardware configuration of the apparatus for determining the possibility of return of spontaneous circulation.
Fig. 3 is a flowchart of a determination program 26.
Figs. 4A and 4B are views showing examples of electrocardiogram waveform data.
Fig. 5 is a view showing examples of electrocardiogram waveform data during chest compression.
Fig. 6 is a view showing examples of electrocardiogram waveform data during chest compression.
Fig. 7 is a view showing examples of electrocardiogram waveform data during chest compression.
Fig. 8 is a view showing examples of electrocardiogram waveform data during chest compression.
Fig. 9 is a functional block diagram of an apparatus which is a second embodiment, and which determines the possibility of return of spontaneous circulation.
Fig. 10 is a flowchart of the determination program 26.

### DETAILED DESCRIPTION OF EXEMPLARY EMBODIMENTS

### 1. First Embodiment

### 1.1 Entire configuration of system

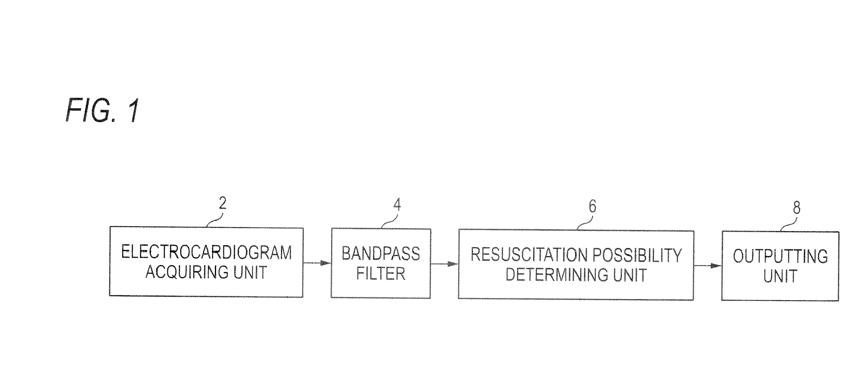

Fig. 1 shows a functional block diagram of an apparatus which is an embodiment of the presently disclosed subject matter, and which determines the possibility of return of spontaneous circulation. An electrocardiogram acquiring unit 2 acquires electrocardiogram waveform data during chest compression which is applied to the subject ins cardiac arrest condition. Since the subject is in an asystole condition or the like, an original electrocardiogram is flat. However, noises due to chest compression can be observed. Here, a waveform containing such noises is called an electrocardiogram waveform.

A bandpass filter 4 receives the acquired electrocardiogram waveform data, and allows only a frequency component in the vicinity of the frequency of the P wave of the received electrocardiogram waveform data to pass therethrough. A resuscitation possibility determining unit 6 determines whether the output of the bandpass filter 4 exceeds a predetermined value or not. If exceeds, it is determined that there is the possibility of return of spontaneous circulation. The outputting unit 8 outputs the result of the determination of the resuscitation possibility determining unit 6.

When the person who performs the chest compression obtains a determination result indicating that there is the possibility of resuscitation, therefore, the person can continue the chest compression. Consequently, the probability of resuscitation can be increased.

### 1.2 Hardware configuration

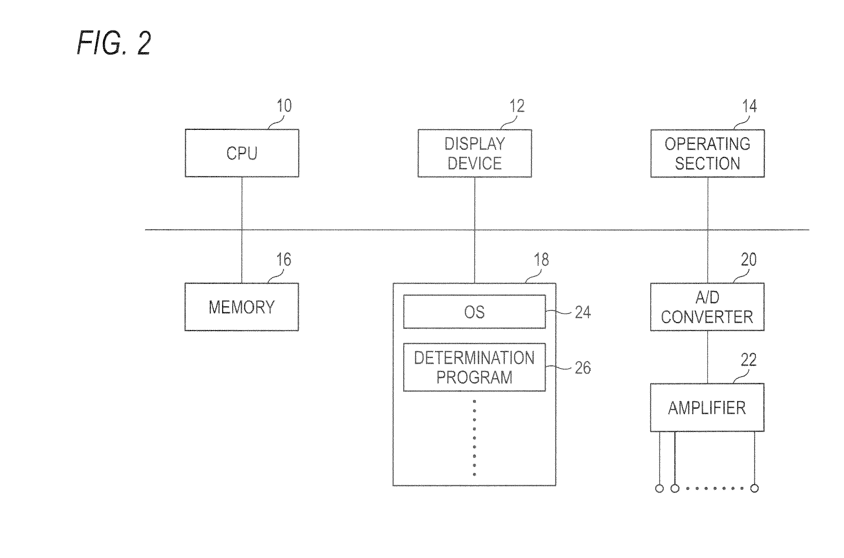

Fig. 2 shows the hardware configuration in the case where the apparatus for determining the possibility of return of spontaneous circulation according to the embodiment of the presently disclosed subject matter is realized by using a CPU 10. A display device 12, an operating section 14, a memory 16, a hard disk drive 18, and an A/D converter 20 are connected to the CPU 10.

ECG electrodes are attached to the body of the subject to acquire electrocardiogram signals of the subject. The electrocardiogram signals from the ECG electrodes are amplified by an amplifier 22, and converted to electrocardiogram waveform signals which are digital data, by the A/D converter 20. The A/D converter 20 accumulates the digital data in the memory 16.

The memory 16 is used as a working area for the CPU 10. The display device 12 displays the determination result and the like. The operating section 14 is configured by buttons and the like through which the operator inputs an operation input. An operating system (for example, WINDOWS (trademark) of Microsoft Corporation) 24, and a determination program 26 for determining the possibility of return of spontaneous circulation are recorded in the hard disk drive 18. The determination program 26 cooperates with the operating system 24 to exert its function. The operating system 24 and determination program 26 which are recorded on a CD-ROM (not shown) are installed in the hard disk drive 18 via a CD-ROM drive (not shown).

### 1.3 Flowchart of determination program

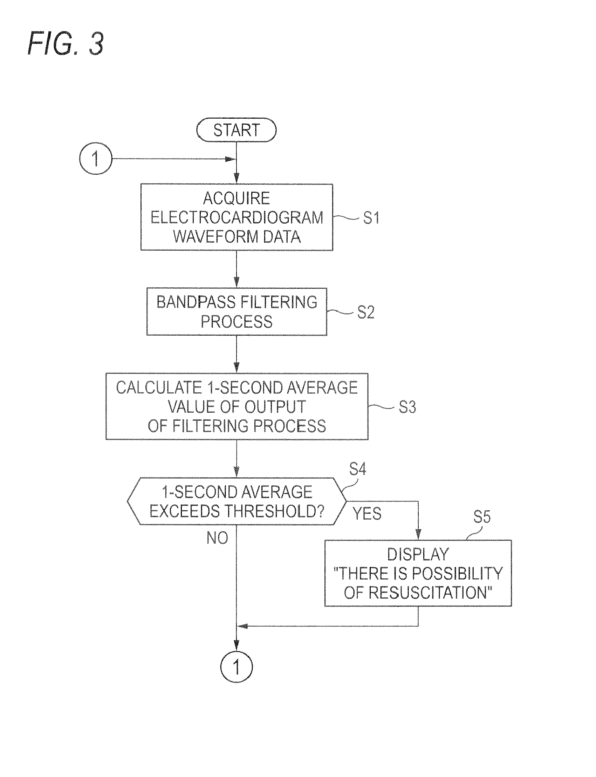

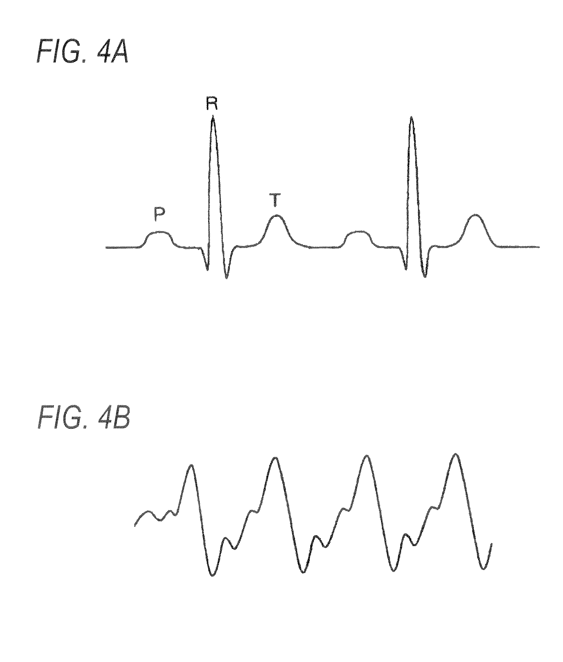

Fig. 3 shows a flowchart of the determination program 26. In step S1, the CPU 10 acquires the electrocardiogram waveform data stored in the memory 16. The electrocardiogram waveforms data are digital data which are obtained by sampling the potential change at predetermined time intervals, and diagrammatically shown in Figs. 4A and 4B.

In the case where the subject is in a normal state, waveform data such as shown in Fig. 4A are acquired, and the P wave, the R wave, the T wave, and the like are observed. By contrast, in the case where chest compression is applied to the subject in a cardiopulmonary arrest condition, electrocardiogram waveform data are as shown in Fig. 4B. Namely, noises due to the chest compression are observed. The intervals of peaks correspond to those of sternal compression of the chest compression.

With respect to each of leads of the acquired electrocardiogram waveform data, the CPU 10 then fetches a component (in the embodiment, 10Hz to 15 Hz) in the vicinity of the frequency of the P wave. In each lead, namely, the bandpass filtering process is performed (step S2).

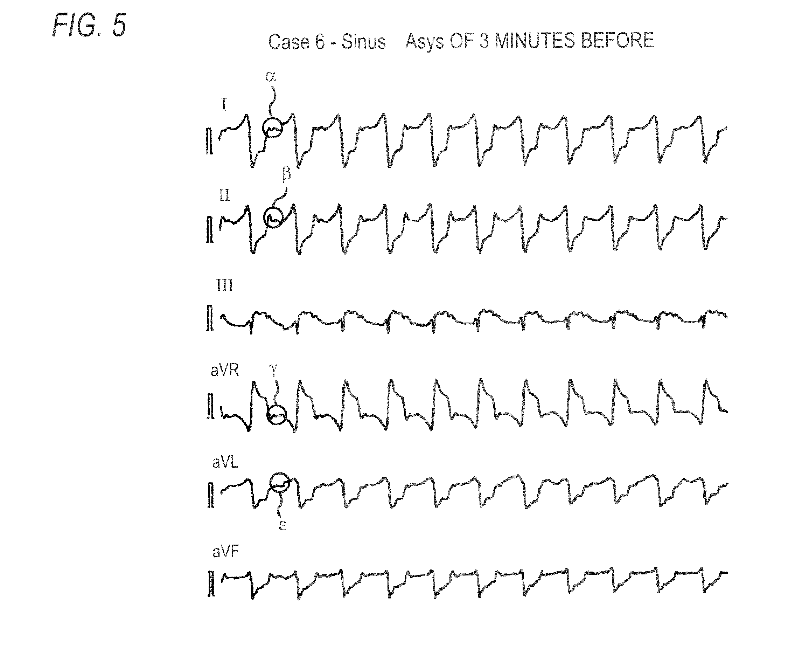

The reason why the bandpass filtering process is performed in the embodiment is as follows. Figs. 5 to 8 show electrocardiogram waveform data which were obtained when chest compression was applied to the subject in a cardiopulmonary arrest condition. The figures show six leads of I, II, III, aver, aVL, and aVF.

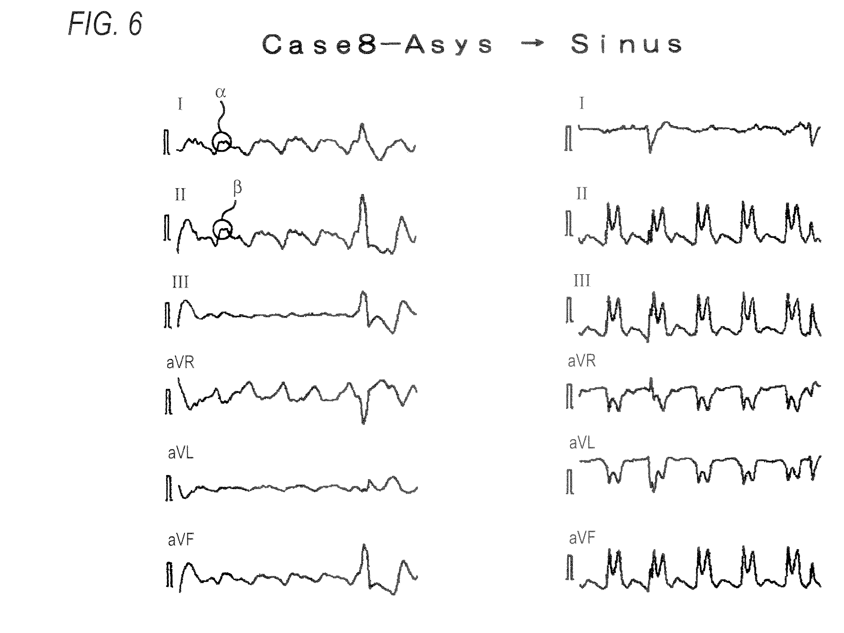

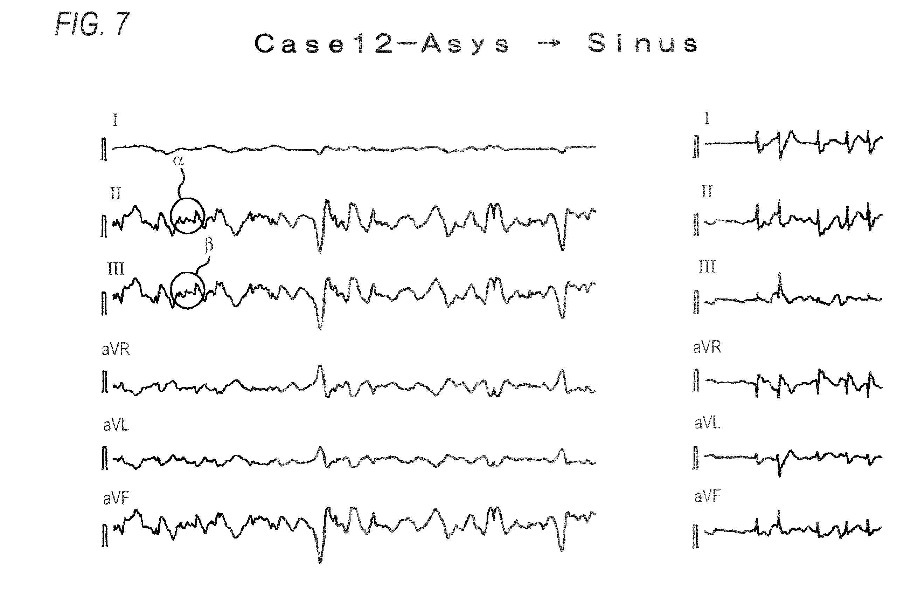

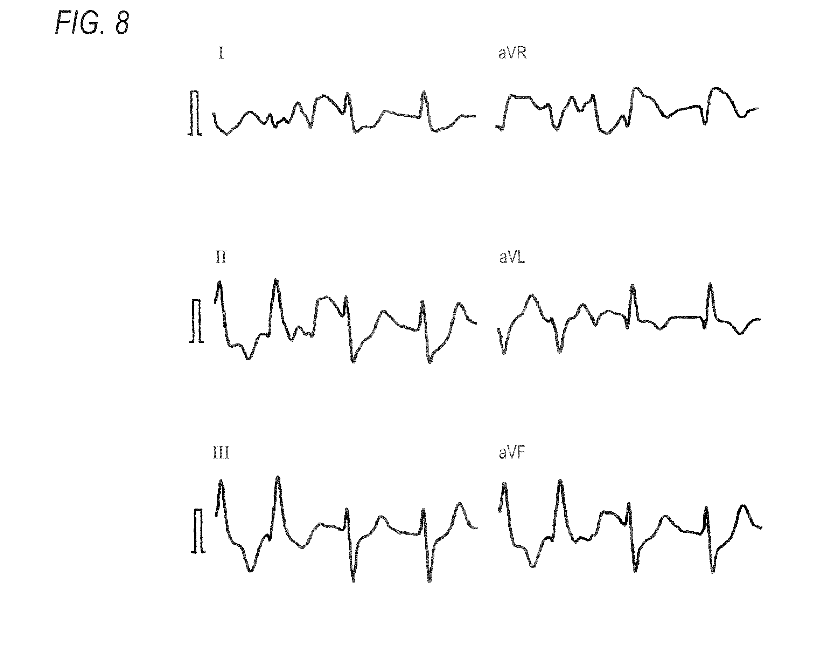

Figs. 5 to 7 show cases where the subject resuscitated as a result of chest compression. Fig. 8 shows a case where the subject did not resuscitate despite chest compression. When the cases are compared to each other, it is seen that, in the cases where resuscitation succeeded, a waveform of a frequency which is higher than that of the intervals of sternal compression appears in any ones of leads of the electrocardiogram waveforms during chest compression. This waveform corresponds to waveforms α, β, γ, ε in Fig. 5, the waveforms α, β in Fig. 6, and the waveforms α, β in Fig. 7. According to experiments conducted by the inventors, it has been proved that the high-frequency components are approximate to the frequency component of the P wave. In Fig. 8, by contrast, such a high-frequency component does not appear.

Namely, in the case where a frequency component in the vicinity of the frequency of the P wave appears in any of leads of electrocardiogram waveforms during chest compression, it is possible to determine that there is the possibility of resuscitation.

In the embodiment, therefore, a frequency component (a component of 10Hz to 15 Hz) in the vicinity of the frequency of the P wave is extracted in each of the leads by the bandpass filter, and its amplitude is measured, thereby enabling the possibility of resuscitation to be determined.

In each of the leads, next, the CPU 10 calculates the average value of the output of the filter over a predetermined time period (in the embodiment, one second) (step S3). Then, the CPU1 10 determines whether the average value of any lead exceeds a predetermined threshold or not (step S4). If not exceeds, the processes subsequent to step S1 are repeated. If exceeds, the CPU 10 causes the display device 12 to display "There is possibility of resuscitation" (step S5).

Therefore, the person who performs the chest compression sees the display of "There is possibility of resuscitation", and can continue the chest compression without hesitation.

The principle of the phenomenon in which, in the cases where resuscitation succeeded, a frequency component in the vicinity of the frequency of the P wave overlaps electrocardiogram waveform data is not clear. For example, it is presumed that the SA channel which is opened and closed by the extension force of chest compression reacts in individual cardiomyoctes, and contraction occurs independently, thereby causing such a frequency component to overlap electrocardiogram waveform data.

### 2. Second Embodiment

### 2.1 Entire configuration of system

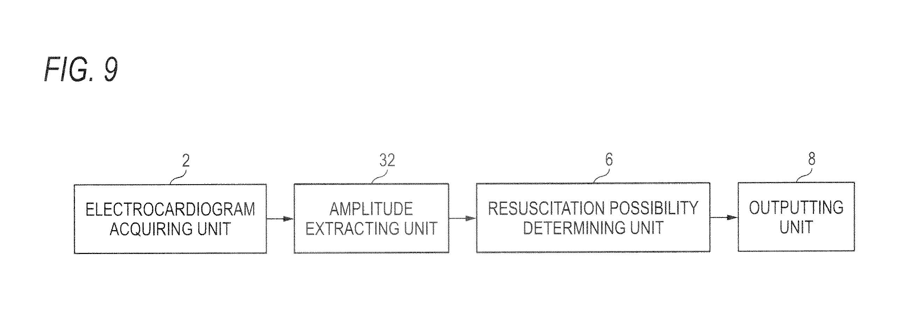

Fig. 9 shows a functional block diagram of an apparatus which is a second embodiment of the presently disclosed subject matter, and which determines the possibility of return of spontaneous circulation. The electrocardiogram acquiring unit 2 acquires electrocardiogram waveform data during chest compression which is applied to the subject in a cardiac arrest condition.

An amplitude extracting unit 32 receives the acquired electrocardiogram waveform data, and extracts the amplitude (for example, the peal value) of the electrocardiogram waveform. The resuscitation possibility determining unit 6 determines whether the amplitude extracted by the amplitude extracting unit 32 is reduced to be lower than that at a reference point (for example, the timing when the chest compression is started) or not. If not reduced to be lower than that at the reference point, it is determined that there is the possibility of return of spontaneous circulation. The outputting unit 8 outputs the result of the determination of the resuscitation possibility determining unit 6.

When the person who performs the chest compression obtains a determination result indicating that there is the possibility of resuscitation, the person can continue the chest compression. Therefore, the probability of resuscitation can be increased.

### 2.2 Hardware configuration

The hardware configuration is similar to that of the first embodiment, but the process contents of the determination program 26 recorded in the hard disk drive 18 is different.

### 2.3 Flowchart of determination program

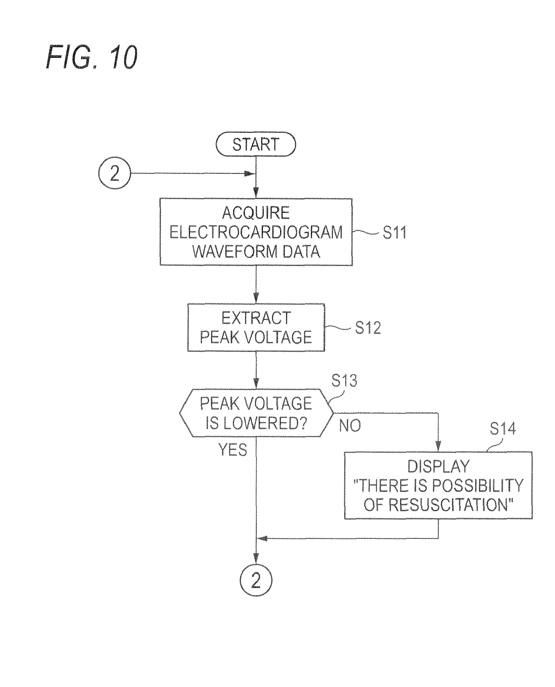

Fig. 10 shows a flowchart of the determination program 26. In step S11, the CPU 10 acquires the electrocardiogram waveform data stored in the memory 16. Fig. 4B diagrammatically shows the acquired data. The intervals of peaks correspond to those of sternal compression of chest compression.

In each of the leads of the acquired electrocardiogram waveform data, next, the CPU 10 calculates the peak voltage for each interval (step S12).

The reason why the extraction of the peak voltage is performed in the embodiment is as follows. Figs. 5 to 8 show electrocardiogram waveform data which were obtained when chest compression was applied to the subject in a cardiopulmonary arrest condition. The figures show six leads of I, II, III, aVR, aVL, and aVF.

Figs. 5 to 7 show cases where the subject resuscitated as a result of chest compression. Fig. 8 shows a case where the subject did not resuscitate despite chest compression.

When the cases are compared to each other, it is seen that, in the cases where resuscitation succeeded (Figs. 5 to 7), the peak value of the electrocardiogram waveform during chest compression is not lowered. By contrast, in the case where resuscitation did not succeed (Fig. 8), the peak value of the electrocardiogram waveform during chest compression is lowered.

Namely, in the case where the peak voltage is raised or maintained in any of leads of electrocardiogram waveform during chest compression, it is possible to determine that there is the possibility of resuscitation.

In the embodiment, therefore, the possibility of resuscitation is determined depending on whether the peak voltage is reduced to be lower than a reference value (for example, the peak value when the chest compression is started, or the average of peak values over a predetermined past time period).

In each of the leads, next, the CPU 10 determines whether the peak voltage is lower than the reference value or not (step S13). If lower, the processes subsequent to step S11 are repeated. If not lower, the CPU 10 causes the display device 12 to display "There is possibility of resuscitation" (step S14).

Therefore, the person who performs the chest compression sees the display of "There is possibility of resuscitation", and can continue the chest compression without hesitation.

The principle of the phenomenon in which, in the case where resuscitation did not succeed, the amplitude of the electrocardiogram waveform itself is lowered is not clear. For example, the possibility is presumed that the amplitude may be lowered by a phenomenon in which muscle controlling emotional sweating relaxes to cause emotional sweating, and the resistance of the skin immediately below the electrodes is reduced.

### 4. Other Embodiments

(1) In the first embodiment, the possibility of resuscitation is determined based on the average value of the output of the bandpass filter in each lead. Alternatively, the determination may be made based on the peak value, waveform integration, or the like of the output of the bandpass filter.
   When any one of the outputs of the leads satisfies the conditions, it is determined that there is the possibility of resuscitation. Alternatively, when the outputs of a plurality of leads satisfy the conditions, it may be determined that there is possibility of resuscitation.
(2) In the second embodiment, when, in any one of the leads, the peak value of the electrocardiogram waveform is not lower than the reference value, it is determined that there is the possibility of resuscitation. Alternatively, when, in all (or a predetermined number or more) of the leads, the peak value of the electrocardiogram waveform is not lower than the reference value, it may be determined that there is the possibility of resuscitation.
(3) Alternatively, the possibility of resuscitation may be determined based on both the output of the bandpass filter and the amplitude of the electrocardiogram waveform itself. When one of the output and the amplitude satisfies the conditions, for example, it may be determined that there is the possibility of resuscitation.
(4) In the embodiments, in the case where, after it is determined that there is the possibility of resuscitation, ventricular fibrillation or pulseless electrical activity is detected, it may be determined that the possibility of resuscitation is increased, and a message indicative this may be displayed. The determination on Ventricular fibrillation or pulseless electrical activity may be made by using a related-art technique.
(5) The embodiments have been described with reference to the case where the apparatus for determining the possibility of return of spontaneous circulation has also the function of acquiring electrocardiogram waveform data, or namely the case where the apparatus is incorporated in an electrocardiograph. Alternatively, the apparatus for determining the possibility of return of spontaneous circulation may be configured separately from an electrocardiograph.
   Alternatively, electrocardiogram waveform data may be transmitted from a terminal apparatus to a server apparatus via the Internet or the like, the server apparatus may determine the possibility of return of spontaneous circulation, and a result of the determination may be returned to the terminal apparatus.
(6) In the embodiments, each of the units is configured by programs. A part or all of the units may be configured by a hardware circuit (for example, a bandpass filter circuit).
   According to an aspect of the presently disclosed subject matter, based on the electrocardiogram waveform during the chest compression, the possibility of resuscitation can be determined, and it is possible to determine whether the chest compression is to be continued or not.

Further, since a frequency component of 10 Hz to 15 Hz is allowed to pass through the bandpass filter, the possibility of resuscitation can be adequately determined.

In embodiments, step S1 in Fig. 3 and step S11 in Fig. 10 correspond to "electrocardiogram acquiring unit."

In the embodiments, step S2 in Fig. 3 corresponds to "bandpass filter."

In the embodiments, step S4 in Fig. 3 and step S13 in Fig. 10 correspond to "resuscitation possibility determining unit."

In the embodiments, step S5 in Fig. 3 and step S14 in Fig. 10 correspond to "outputting unit."

"Program" has a concept including not only a program which is directly executed by a CPU, but also a source format program, a compressed program, an encrypted program, and the like.

## Claims

1. An apparatus for determining a possibility of return of spontaneous circulation, the apparatus comprising:
an electrocardiogram acquiring unit which is configured to acquire electrocardiogram waveform data from a subject in a cardiac arrest condition during chest compression which is applied to the subject as a cardiopulmonary resuscitation process;
a bandpass filter unit which is configured to receive the acquired electrocardiogram waveform data, and which is configured to allow only a frequency component in a vicinity of a frequency of a P wave of the received electrocardiogram waveform to pass through the bandpass filter unit;
a resuscitation possibility determining unit which is configured to determine whether an output of the bandpass filter unit exceeds a predetermined value or not, and which is configured to determine that there is the possibility of return of spontaneous circulation when the output exceeds the predetermined value; and
an outputting unit which is configured to output a result of determination related to the possibility of return of spontaneous circulation.

2. The apparatus according to claim 1, wherein
the frequency component allowed to pass the bandpass filter unit is a frequency component of 10 Hz to 15 Hz.

3. A method of determining a possibility of return of spontaneous circulation, the method comprising:
acquiring electrocardiogram waveform data from a subject in a cardiac arrest condition during chest compression which is applied to the subject as a cardiopulmonary resuscitation process;
receiving the acquired electrocardiogram waveform data, allowing only a frequency component in a vicinity of a frequency of a P wave of the received electrocardiogram waveform to pass through a bandpass filter;
determining whether an output of the bandpass filter exceeds a predetermined value or not, and determining that there is the possibility of return of spontaneous circulation when the output exceeds the predetermined value; and
outputting a result of determination related to the possibility of return of spontaneous circulation.

4. The method according to claim 3, wherein
the frequency component allowed to pass the bandpass filter is a frequency component of 10 Hz to 15 Hz.

5. A non-transitory computer-readable recording medium in which a computer program causing a computer to execute the method according to claim 3 is recorded.

6. The non-transitory computer-readable recording medium according to claim 5, wherein
the frequency component allowed to pass the bandpass filter is a frequency component of 10 Hz to 15 Hz.

7. An apparatus for determining a possibility of return of spontaneous circulation, the apparatus comprising:
an electrocardiogram acquiring unit which is configured to acquire electrocardiogram waveform data from a subject in a cardiac arrest condition during a cardiopulmonary resuscitation process applied to the subject;
a resuscitation possibility determining unit which is configured to receive the acquired electrocardiogram waveform data, and which is configured to determine that there is a possibility of return of spontaneous circulation when an amplitude of the received electrocardiogram waveform data is not lower than a reference value; and
an outputting unit which is configured to output a result of the determination.

8. A method of determining a possibility of return of spontaneous circulation, the method comprising:
acquiring electrocardiogram waveform data from a subject in a cardiac arrest condition during a cardiopulmonary resuscitation process applied to the subject;
receiving the acquired electrocardiogram waveform data, and determining that there is a possibility of return of spontaneous circulation when an amplitude of the received electrocardiogram waveform data is not lower than a reference value; and
outputting a result of the determination,

9. A non-transitory computer-readable recording medium in which a computer program causing a computer to execute the method according to claim 8 is recorded.
